(19) Europäisches Patentamt / European Patent Office / Office européen des brevets

(11) **EP 4 122 473 A1**

(12) **DEMANDE DE BREVET EUROPEEN**

(43) Date de publication:
**25.01.2023 Bulletin 2023/04**

(21) Numéro de dépôt: **22185681.8**

(22) Date de dépôt: **19.07.2022**

(51) Classification Internationale des Brevets (IPC):
*A61K 33/44* (2006.01)  *A61D 7/00* (2006.01)
*A61K 9/00* (2006.01)  *A61K 9/10* (2006.01)
*A61K 47/10* (2017.01)  *A61K 47/36* (2006.01)
*A61P 1/00* (2006.01)  *A61P 39/00* (2006.01)
*A61M 5/315* (2006.01)

(52) Classification Coopérative des Brevets (CPC):
**A61K 33/44; A61D 7/00; A61K 9/0056; A61K 9/10; A61K 47/10; A61K 47/36; A61P 1/00; A61P 39/00;** A61M 5/31528; A61M 5/31551

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **19.07.2021 FR 2107759**

(71) Demandeur: **Domes Pharma**
**63430 Pont-du-Château (FR)**

(72) Inventeur: **CHAUDER, Anne**
**63430 PONT-DU-CHÂTEAU (FR)**

(74) Mandataire: **Cabinet Laurent & Charras**
**Le Contemporain**
**50 Chemin de la Bruyère**
**69574 Dardilly Cedex (FR)**

(54) **COMPOSITION A BASE DE CHARBON ACTIF UTILISEE DANS LE TRAITEMENT DES TROUBLES DIGESTIFS CHEZ LES ANIMAUX**

(57) La présente invention concerne une composition comprenant du charbon actif pour le traitement d'un trouble digestif chez un animal, caractérisée en ce que la composition est sous une forme adaptée pour l'administration par voie orale à l'aide d'une seringue (1).

FIGURE 1

EP 4 122 473 A1

**Description**

**Domaine technique de l'invention**

La présente invention se rapporte à une composition à base de charbon actif utilisée dans le traitement des troubles digestifs, notamment des intoxications par voie orale, chez les animaux.

**Arrière-plan technologique**

Dans le domaine du traitement des troubles digestifs chez les animaux, en particulier des intoxications par voie orale, il est connu d'utiliser des compositions administrées par voie orale, comprenant un agent adsorbant dont le rôle est de capter les substances toxiques par adsorption de ces dernières.

Les compositions connues et couramment utilisées dans le domaine se présentent typiquement sous la forme de poudre, de granulés, ou de suspension, généralement contenues dans un récipient de grand volume tel qu'un sceau.

A ce sujet, le document US 3917821 décrit un procédé d'administration de charbon actif pour adsorber des substances toxiques préalablement ingérées. Le charbon actif sous forme de poudre est combiné à un polysaccharide épaississant et soluble dans l'eau. La composition destinée à être administrée se présente alors sous la forme d'une pâte (« aqueous slurry » en anglais).

Le conditionnement des compositions sous forme de poudre ou de granulés dans des récipients de grand volume est adapté pour les animaux de rente, tels que les veaux, les agneaux, les chevaux, ou encore les poneys, mais ne convient pas pour traiter des animaux de compagnie tels que des chiens et des chats. En effet, le traitement des animaux de compagnie nécessite seulement de faibles volumes de composition, et le nombre d'itérations du traitement reste généralement limité.

Les suspensions liquides peuvent être conditionnées dans des flacons. On utilise une seringue pour prélever la suspension du flacon et l'administrer à l'animal, le plus souvent par voie orale.

Cependant, prélever la suspension à l'aide d'une seringue, et ce pour chaque animal à traiter, peut se révéler fastidieux et prendre du temps, notamment lorsque le nombre d'animaux à traiter est important. Il en est de même pour le prélèvement des poudres et des granulés à l'aide d'une pelle doseuse.

Cette étape de préparation prend un temps non négligeable, qui peut s'avérer critique dans le cas d'une situation d'urgence où il est nécessaire d'administrer le plus rapidement possible la composition à l'animal sous peine de complications.

Par ailleurs, les flacons contenant les compositions sont généralement fournis sans seringue, de sorte que l'utilisateur doit en parallèle se procurer des seringues, qui en outre doivent être adaptées à l'administration de ce type de composition.

En ce sens, le document BURKITT JAMIE M. ET AL : « Effects of Oral Administration of a Commercial Activated Charcoal Suspension on Serum Osmolality and Lactate Concentration in The Dog », JOURNAL OF VETERINARY INTERNAL MEDICINE, Vol.19, no. 5, 1 septembre 2005 (2005-09-01), pages 683-686 (XP55898732) décrit à titre expérimental une composition pour traiter l'intoxication intestinale du chien sous la forme d'une suspension combinant du charbon actif avec du glycérol et propylène glycol en tant qu'agent stabilisant. La composition est administrée en la versant dans le bol ou, en cas de refus, au moyen d'une seringue ou d'une sonde.

Le document WO2004/043453A1 décrit une seringue permettant d'administrer un mélange à base de lysine au chat pour le traitement des infections virales du type herpes (FHV-1). La lysine est solubilisée au préalable dans l'eau. Il est indiqué que le mélange est conditionné et/ou administré avec une seringue.

**Description de l'invention**

Un but de l'invention est de proposer une composition permettant de surmonter les inconvénients mentionnés précédemment.

L'invention vise tout particulièrement à fournir une telle composition comprenant un agent adsorbant apte à adsorber des substances toxiques présentes dans le système digestif d'un animal, dans le cadre d'un traitement d'un trouble digestif, notamment d'une intoxication par voie orale, chez un animal.

L'invention vise tout particulièrement à fournir une telle composition, permettant de s'affranchir des étapes de préparation et de conditionnement de la composition, notamment le fait de devoir prélever la composition dans un récipient à l'aide d'une seringue et de devoir doser précisément la quantité à administrer. Ceci permet alors de gagner du temps et d'éviter les incidents pouvant survenir lors de ces étapes de préparation et de conditionnement de la composition.

A cette fin, l'invention propose une composition comprenant du charbon actif pour le traitement d'un trouble digestif chez un animal, principalement caractérisée en ce que la composition est sous une forme adaptée pour l'administration par voie orale à l'aide d'une seringue.

De préférence, le trouble digestif destiné à être traité par la composition est une intoxication par voie orale.

Une intoxication par voie orale comprend par exemple une intoxication médicamenteuse, une intoxication alimentaire, une intoxication par ingestion de plantes, de raticides, ou encore de nicotine via une cigarette ou de la gomme à mâcher par exemple.

L'invention se rapporte également à une composition conditionnée dans une seringue prête à l'emploi, ladite composition comprenant du charbon actif.

La composition est conditionnée dans une seringue qui est dite « prête à l'emploi », en ce que la seringue est pré-remplie avec la composition. L'utilisateur peut alors directement administrer la composition contenue dans la seringue à l'animal par voie orale, sans devoir préparer la composition ou prélever la composition depuis un contenant distinct de la seringue. Ceci réduit les manipulations nécessaires au traitement, rendant ce dernier plus simple, plus sûr, et plus rapide. On comprend donc que le terme « prête à l'emploi » s'applique à la seringue contenant la composition à administrer et stockée telle quel, avant usage sans que la stabilité de la composition ne soit affectée. Le stockage peut excéder plusieurs mois, voire plusieurs années.

Les animaux concernés sont préférentiellement les animaux de compagnie tels que chiens et chats par exemple.

La quantité de composition contenue dans la seringue ainsi que les concentrations des différents constituants, sont ajustés en fonction du type d'animal à traiter, et éventuellement d'autres paramètres tels que la durée du traitement ou la gravité de l'intoxication par voie orale.

La seringue comprend typiquement un réservoir cylindrique contenant la composition, et une tige de piston apte à coulisser longitudinalement dans le réservoir pour administrer la composition.

La seringue est dépourvue d'aiguille d'injection, celle-ci étant inutile puisque l'administration est réalisée par voie orale et non par voie parentérale. En outre, une telle aiguille pourrait représenter un risque de blessure pour l'animal comme pour l'utilisateur. On préférera donc une seringue dans laquelle la partie distale du réservoir comprend un embout tubulaire en communication avec ledit réservoir permettant d'acheminer la composition hors du réservoir.

Dans le présent texte, l'extrémité distale de la seringue correspond à l'extrémité la plus éloignée de la main de l'utilisateur (extrémité de l'embout tubulaire), et l'extrémité proximale correspond à l'extrémité la plus proche de la main de l'utilisateur (extrémité opposée à l'embout tubulaire). Ainsi, la direction distale est la direction d'administration de la composition, et la direction proximale est la direction opposée, c'est-à-dire la direction vers la main de l'utilisateur.

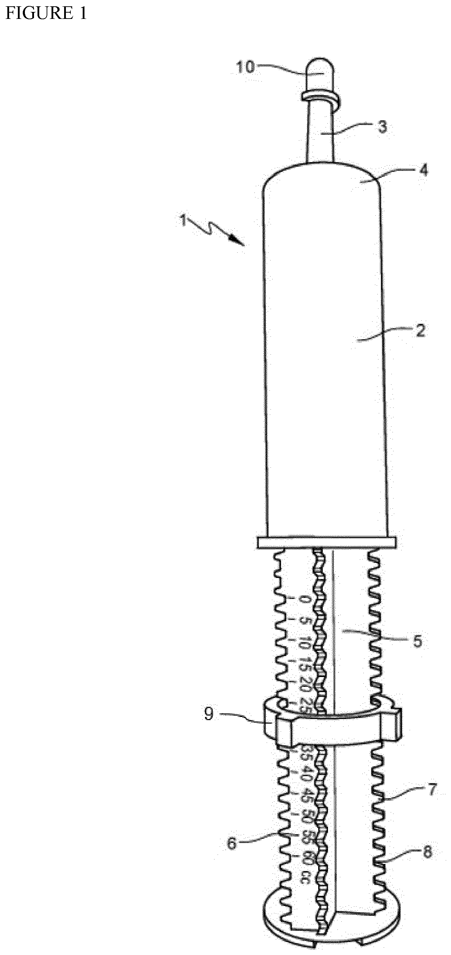

Selon un mode de réalisation préféré, illustré sur la figure 1, la seringue 1 comprend :

- un réservoir 2 contenant la composition,
- un embout tubulaire 3 agencé à une extrémité distale 4 du réservoir et en communication avec ledit réservoir,
- une tige de piston 5 apte à coulisser dans le réservoir 2 en direction distale afin d'expulser la composition hors du réservoir via l'embout tubulaire 3, ladite tige de piston 5 comprenant une graduation 6 du volume de composition à administrer et une alternance de dents 7 et de crans 8,
- une bague de réglage 9, apte à se déplacer par translation-rotation le long de la tige de piston 5, afin de définir un volume de composition à administrer en correspondance avec la graduation.

La seringue 1 peut en outre comprendre un bouchon 10 apte à recouvrir l'embout tubulaire 3, afin d'empêcher tout déversement intempestif de la composition hors de la seringue via ledit embout tubulaire, lors du stockage de la seringue. Le bouchon 10 permet avantageusement une utilisation répétée de la seringue, c'est-à-dire plusieurs administrations de fractions de la composition espacées dans le temps (utilisation multi-doses).

La seringue est avantageusement stockée et commercialisée pré-remplie, et la bague de réglage permet, par vissage/dévissage selon un mouvement hélicoïdal, de régler le volume de composition à administrer. Il peut s'agit du volume total de composition contenu dans la seringue, auquel cas la bague sera positionnée à l'extrémité proximale de la tige de piston en correspondance avec la graduation la plus élevée, ou d'une partie seulement du volume total de composition, auquel cas la bague sera positionnée au niveau d'une graduation intermédiaire.

La seringue peut être à usage unique (mono-dose), ou bien réutilisable (multi-doses).

Le charbon actif est un agent adsorbant antitoxique permettant de fixer les substances toxiques du système digestif de l'animal par adsorption, et évite ainsi le passage de ceux-ci dans le système systémique.

De préférence, la composition comprend de 20% à 60% en poids de charbon actif, par rapport au poids total de la composition. Cette gamme de concentration en charbon actif permet d'obtenir un bon effet antitoxique, tout en rendant compatible la composition avec son administration dans une seringue par voie orale.

Selon un mode de réalisation, la composition comprend :

- de 20% à 60% en poids de charbon actif,
- de 10% à 34% en poids de glycérine,
- de 0,10% à 1,25% en poids de gomme de xanthane,

- de 25% à 70% en poids d'eau.

Les concentrations, en pourcentages massiques, sont exprimées par rapport au poids total de la composition.

La glycérine (ou glycérol) et la gomme de xanthane présentent un pouvoir épaississant et suspensif. Leur pouvoir suspensif permet à la composition de prendre la forme d'une suspension (liquide) ou d'une pâte, en fonction de la concentration de glycérine et de gomme de xanthane, ainsi que de la nature et des concentrations massiques des autres constituants de la composition, dans laquelle le charbon actif (initialement sous forme d'une poudre), la glycérine, et la gomme de xanthane sont sous forme de fines particules dispersées dans la phase aqueuse. La stabilité de cette suspension ou de cette pâte est très peu affectée par la température, et la suspension ou la pâte est parfaitement stable à la température du corps de l'animal qui se situe généralement entre 38°C et 39°C, voire quelques degrés de plus en cas de fièvre.

Le pouvoir épaississant de la glycérine et de la gomme de xanthane permet d'ajuster la viscosité de la composition à une valeur souhaitée, pour la rendre compatible avec une administration par voie orale via une seringue. Par ailleurs, contrairement à la plupart des gommes naturelles, la gomme de xanthane est stable en milieu acide, assurant ainsi la stabilité de la composition en présence des sucs gastriques dans l'estomac de l'animal.

Le Demandeur a découvert que les gammes de concentration massiques de 20% à 60% de charbon actif, de 10% à 34% de glycérine, de 0,10% à 1,25% de gomme de xanthane, et de 25% à 70% d'eau, permettent avantageusement d'obtenir un traitement suffisamment rapide et efficace pour que le charbon actif puisse fixer les toxiques, et compatible avec un conditionnement de la composition dans une seringue pour une administration par voie orale, notamment du fait que la viscosité de la composition est suffisamment basse pour ne pas entraver son administration via la seringue.

La viscosité de la composition dépend de la forme mise en œuvre, qui peut être liquide ou pâteuse. Ce point sera expliqué plus en détail dans la suite du présent texte.

La viscosité peut être mesurée par exemple selon la méthode décrite selon la pharmacopée européenne, notamment dans le document « *European Pharmacopeia 10.0* », parts 2.2.8. Viscosity and 2.2.9. Capillary Viscosimeter Method.

La viscosité correspond à la viscosité dynamique, usuellement notée $\eta$. La détermination de la viscosité est réalisée avec un viscosimètre capillaire à niveau suspendu (de type Ubbelohde) à une température de 20.0°C $\pm$ 0,1°C. Le temps requis pour que le niveau de liquide passe d'une marque à l'autre est mesuré.

Toujours selon ce mode de réalisation, la composition est de préférence constituée ou essentiellement constituée de charbon actif, glycérine, gomme de xanthane, et d'eau. Dans ce cas, la somme des pourcentages massiques en charbon actif, glycérine, gomme de xanthane, et en eau est égale ou environ égale à 100%.

De manière générale, et de préférence, la composition ne comprend pas d'autre agent adsorbant que le charbon actif.

La composition selon l'invention peut se présenter sous la forme d'un liquide ou d'une pâte, en fonction des concentrations massiques en charbon actif, glycérine, gomme de xanthane, et en eau.

Selon un mode de réalisation, la composition se présente sous la forme liquide, et comprend, par rapport au poids total de la composition :

- de 20% à 60% en poids de charbon actif, de préférence de 20% à 35% en poids,
- de 10% à 34% en poids de glycérine, de préférence de 15% à 25% en poids,
- de 0,10% à 0,50% en poids de gomme de xanthane, de préférence de 0,15% à 0,20% en poids,
- de 25% à 70% en poids d'eau.

Ces gammes de concentration resserrées permettent d'obtenir une composition liquide dont la stabilité dans le temps est optimale, tout en conservant une viscosité adaptée à une administration par voie orale via une seringue.

La viscosité de la composition liquide, mesurée à une température de 20°C, est de préférence comprise entre 500 mPa.s et 1500 mPa.s, de préférence entre 800 mPa.s et 1200 mPa.s, et plus préférentiellement entre 900 mPa.s et 1000 mPa.s.

Selon un mode de réalisation, la composition se présente sous la forme d'une pâte, et comprend, par rapport au poids total de la composition :

- de 20% à 60% en poids de charbon actif, de préférence de 20% à 35% en poids,
- de 10% à 34% en poids de glycérine, de préférence de 15% à 25% en poids,
- de 0,50% à 1% en poids de gomme de xanthane, de préférence de 0,60% à 0,90% en poids, et plus préférentiellement de 0,70% à 0,80% en poids,
- de 25% à 70% en poids d'eau.

Comme mentionné précédemment, la glycérine et la gomme de xanthane présentent un pouvoir épaississant et suspensif permettant à la composition de prendre la forme d'une suspension (liquide) ou d'une pâte, en fonction de leur concentration, ainsi que de la nature et des concentrations massiques des autres constituants de la composition. Cela signifie que deux compositions pourront par exemple comprendre une concentration identique de gomme de xanthane, la concentration des autres constituants, en particulier de la glycérine, déterminant leur caractère liquide ou pâteux.

Ces gammes de concentration resserrées permettent d'obtenir une composition pâteuse dont la stabilité dans le temps est optimale, tout en conservant une viscosité adaptée à une administration par voie orale via une seringue.

La viscosité de la composition pâteuse, mesurée à une température de 20°C, est de préférence comprise entre 5000 mPa.s et 20000 mPa.s, de préférence entre 10000 mPa.s et 15000 mPa.s, et plus préférentiellement entre 11000 mPa.s et 13000 mPa.s.

Le terme « pâte », relatif à la composition de l'invention, correspond à un mélange visqueux présentant une viscosité telle que décrite ci-dessus. Cette définition englobe également un gel qui correspond à un réseau solide au sein d'un fluide.

Par ailleurs, la composition comprend avantageusement au moins un conservateur antimicrobien. Le conservateur antimicrobien est de préférence choisi parmi : sorbate de potassium, ammonium quaternaire (chlorure de benzalkonium (acronyme CBK) ou cétrimide par exemple), dérivé alcoolique (alcool benzylique, chlorobutanol, bronopol, ou phénol par exemple), dérivé acide (acide benzoïque, acide sorbique, benzoate de sodium par exemple), chlorhexidine, paraben, mercuriel, et leurs mélanges.

De préférence, la composition comprend du sorbate de potassium en tant que conservateur antimicrobien selon une quantité allant de 0,5% à 2% en poids, de préférence de 0,5% à 1,5% en poids, par rapport au poids total de la composition.

De préférence, la composition est constituée ou essentiellement constituée de charbon actif, glycérine, gomme de xanthane, sorbate de potassium, et d'eau. La somme des pourcentages massiques des constituants est alors égale à 100%.

La composition comprend avantageusement au moins un agent conservateur antioxydant. Le conservateur antioxydant est de préférence choisi parmi : bisulfite de sodium, benzoate de sodium, EDTA, et leurs mélanges.

La composition présente avantageusement un pouvoir adsorbant supérieur ou égal à 8%, c'est-à-dire 8 g/100 g. La composition sous forme liquide présente de préférence un pouvoir adsorbant supérieur ou égal à 8% lorsque la concentration en charbon actif est d'au moins 20% en poids. La composition sous forme pâteuse présente de préférence un pouvoir adsorbant supérieur ou égal à 12% lorsque la concentration en charbon actif est d'au moins 30% en poids.

Le pouvoir adsorbant est déterminé par la mesure de l'indice de phénazone d'un mélange comprenant ladite composition et de la phénazone, la quantité en de phénazone adsorbée étant calculé avec la formule suivante (1) :
[Math. 1]

$$Q = 2{,}353*(V_b - V) / m \qquad (1)$$

dans laquelle,

- Q est la quantité de phénazone adsorbée en gramme pour 100g de composition,
- m est la masse de la prise d'essai en gramme,
- $V_b$ est le volume en ml de bromate de potassium 0.0167M versé dans l'essai à blanc,
- V est le volume en ml de bromate de potassium 0.0167M versé dans l'essai.

**Description des figures**

[Fig. 1] illustre un mode de réalisation préféré et non limitatif, d'une seringue prête à l'emploi pour administrer une composition à base de charbon actif, selon l'invention.

Exemple 1 :

On prépare une composition sous la forme d'une pâte ayant la formule suivante :

- de 20% à 35% en poids de charbon actif, ,
- de 15% à 25% en poids de glycérine,
- de 0,70% à 0,8% en poids de gomme de xanthane, d,
- de 25% à 70% en poids d'eau.

On remplit une seringue de 60 ml avec cette composition puis on vérifie la stabilité de la composition, en particulier la viscosité à 6 et 8 mois de stockage.

La viscosité est mesurée selon la méthode décrite selon la pharmacopée européenne, dans le document « European Pharmacopeia 10.0 », parts 2.2.8. Viscosity and 2.2.9. Capillary Viscosimeter Method mentionné précédemment.

Les résultats figurent dans le tableau suivant :

| | 6 MOIS 25°C 40% d'humidité relative | 6 MOIS 40 °C 40% d'humidité relative | 12MOIS 25°C 40% d'humidité relative |
|---|---|---|---|
| Viscosité mPa.s 5 000 à 20 000 | 12 480 | 11 510 | 15 490 |

## Revendications

1. Composition comprenant du charbon actif pour le traitement d'un trouble digestif chez un animal, sous une forme adaptée pour l'administration par voie orale, **caractérisée en ce qu'**elle est conditionnée dans une seringue (1) préremplie et prête à l'emploi.

2. Composition selon la revendication 1, **caractérisée en ce qu'**elle comprend de 20% à 60% en poids de charbon actif, par rapport au poids total de la composition.

3. Composition selon la revendication 1, **caractérisée en ce qu'**elle comprend, par rapport au poids total de la composition :

   - de 20% à 60% en poids de charbon actif,
   - de 10% à 34% en poids de glycérine,
   - de 0,10% à 1,25% en poids de gomme de xanthane,
   - de 25% à 70% en poids d'eau.

4. Composition selon l'une quelconque des revendications 1 à 3, **caractérisée en ce qu'**elle se présente sous la forme d'un liquide, et comprend, par rapport au poids total de la composition :

   - de 20% à 60% en poids de charbon actif, de préférence de 20% à 35% en poids,
   - de 10% à 34% en poids de glycérine, de préférence de 15% à 25% en poids,
   - de 0,10% à 0,50% en poids de gomme de xanthane, de préférence de 0,15% à 0,20% en poids,
   - de 25% à 70% en poids d'eau.

5. Composition selon l'une quelconque des revendications 1 à 3, **caractérisée en ce qu'**elle se présente sous la forme d'une pâte, et comprend, par rapport au poids total de la composition :

   - de 20% à 60% en poids de charbon actif, de préférence de 20% à 35% en poids,
   - de 10% à 34% en poids de glycérine, de préférence de 15% à 25% en poids,
   - de 0,50% à 1% en poids de gomme de xanthane, de préférence de 0,60% à 0,90% en poids, et plus préférentiellement de 0,70% à 0,80% en poids,
   - de 25% à 70% en poids d'eau.

6. Composition selon l'une quelconque des revendications 1 à 3 et 5, **caractérisée en ce qu'**elle se présente sous la forme d'une pâte, et présente une viscosité à 20°C comprise entre 5000 mPa.s et 20000 mPa.s, de préférence entre 10000 mPa.s et 15000 mPa.s, et plus préférentiellement entre 11000 mPa.s et 13000 mPa.s.

7. Composition selon l'une quelconque des revendications précédentes, **caractérisée en ce qu'**elle comprend en outre du sorbate de potassium en tant que conservateur antimicrobien selon une quantité allant de 0,5% à 2% en poids, de préférence de 0,5% à 1,5% en poids, par rapport au poids total de la composition.

8. Composition selon l'une quelconque des revendications précédentes, **caractérisée en ce que** la seringue (1) comprend :

- un réservoir (2) contenant la composition,
- un embout tubulaire (3) agencé à une extrémité distale (4) du réservoir et en communication avec ledit réservoir,
- une tige de piston (5) apte à coulisser dans le réservoir (2) en direction distale afin d'expulser la composition hors du réservoir via l'embout tubulaire (3), ladite tige de piston (5) comprenant une graduation (6) du volume de composition à administrer et une alternance de dents (7) et de crans (8),
- une bague de réglage (9), apte à se déplacer par translation-rotation le long de la tige de piston (5), afin de définir un volume de composition à administrer en correspondance avec la graduation.

9. Composition conditionnée dans une seringue (1) prête à l'emploi, ladite composition étant **caractérisée en ce qu'**elle comprend, par rapport au poids total de la composition :

- de 20% à 60% en poids de charbon actif,
- de 10% à 34% en poids de glycérine,
- de 0,10% à 1,25% en poids de gomme de xanthane,
- de 25% à 70% en poids d'eau.

10. Composition conditionnée dans une seringue (1) prête à l'emploi selon la revendication 9, ladite composition étant **caractérisée en ce qu'**elle se présente sous la forme d'un liquide, et comprend, par rapport au poids total de la composition :

- de 20% à 60% en poids de charbon actif, de préférence de 20% à 35% en poids,
- de 10% à 34% en poids de glycérine, de préférence de 15% à 25% en poids,
- de 0,10% à 0,50% en poids de gomme de xanthane, de préférence de 0,15% à 0,20% en poids,
- de 25% à 70% en poids d'eau.

11. Composition conditionnée dans une seringue (1) prête à l'emploi selon l'une quelconque des revendications 9 et 10, ladite composition étant **caractérisée en ce qu'**elle se présente sous la forme d'un liquide, et présente une viscosité à 20°C comprise entre 500 mPa.s et 1500 mPa.s, de préférence entre 800 mPa.s et 1200 mPa.s, et plus préférentiellement entre 900 mPa.s et 1000 mPa.s.

12. Composition conditionnée dans une seringue (1) prête à l'emploi selon la revendication 9, ladite composition étant **caractérisée en ce qu'**elle se présente sous la forme d'une pâte, et comprend, par rapport au poids total de la composition :

- de 20% à 60% en poids de charbon actif, de préférence de 20% à 35% en poids,
- de 10% à 34% en poids de glycérine, de préférence de 15% à 25% en poids,
- de 0,50% à 1% en poids de gomme de xanthane, de préférence de 0,60% à 0,90% en poids, et plus préférentiellement de 0,70% à 0,80% en poids,
- de 25% à 70% en poids d'eau.

13. Composition conditionnée dans une seringue (1) prête à l'emploi selon l'une quelconque des revendications 9 et 12, ladite composition étant **caractérisée en ce qu'**elle se présente sous la forme d'une pâte, et présente une viscosité à 20°C comprise entre 5000 mPa.s et 20000 mPa.s, de préférence entre 10000 mPa.s et 15000 mPa.s, et plus préférentiellement entre 11000 mPa.s et 13000 mPa.s.

14. Composition conditionnée dans une seringue (1) prête à l'emploi selon l'une quelconque des revendications 9 à 13, ladite composition étant **caractérisée en ce qu'**elle comprend en outre du sorbate de potassium en tant que conservateur antimicrobien selon une quantité allant de 0,5% à 2% en poids, de préférence de 0,5% à 1,5% en poids, par rapport au poids total de la composition.

15. Composition conditionnée dans une seringue (1) prête à l'emploi selon l'une quelconque des revendications 9 à 14, ladite composition étant **caractérisée en ce que** la seringue (1) comprend :

- un réservoir (2) contenant la composition,
- un embout tubulaire (3) agencé à une extrémité distale (4) du réservoir et en communication avec ledit réservoir,
- une tige de piston (5) apte à coulisser dans le réservoir (2) en direction distale afin d'expulser la composition hors du réservoir via l'embout tubulaire (3), ladite tige de piston (5) comprenant une graduation (6) du volume de composition à administrer et une alternance de dents (7) et de crans (8),
- une bague de réglage (9), apte à se déplacer par translation-rotation le long de la tige de piston (5), afin de définir un volume de composition à administrer en correspondance avec la graduation.

FIGURE 1

# EP 4 122 473 A1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | SCHOLTZ E C ET AL: "EVALUATION OF FIVE ACTIVATED CHARCOAL FORMULATION FOR INHIBITION OFASPIRIN ABSORPTION AND PALATABILITY IN MAN", AMERICAN JOURNAL OF HOSPITAL PHARMACY, AMERICAN SOCIETY OF HOSPITAL PHARMACISTS, BETHESDA, MD, US, vol. 35, no. 11, 1 novembre 1978 (1978-11-01), pages 1355-1359, XP000564979, ISSN: 0002-9289 | 1,2 | INV. A61K33/44 A61D7/00 A61K9/00 A61K9/10 A61K47/10 A61K47/36 A61P1/00 A61P39/00 A61M5/315 |
| Y | * abrégé * * page 1355, colonne de gauche, alinéa 1 * * page 1356, colonne de gauche, alinéa 1 – colonne de droite, alinéa 1 * ----- | 1-15 | |
| X | BURKITT JAMIE M. ET AL: "Effects of Oral Administration of a Commercial Activated Charcoal Suspension on Serum Osmolality and Lactate Concentration in the Dog", JOURNAL OF VETERINARY INTERNAL MEDICINE, vol. 19, no. 5, 1 septembre 2005 (2005-09-01), pages 683-686, XP055898732, US ISSN: 0891-6640, DOI: 10.1111/j.1939-1676.2005.tb02746.x | 1 | |
| Y | * abrégé * * page 683, colonne de gauche, alinéa 1-5 * * page 683, colonne de droite, alinéa 4 * * page 684, colonne de gauche, alinéa 2 * ----- -/-- | 1-15 | |

**DOMAINES TECHNIQUES RECHERCHES (IPC)**

A61D
A61K
A61P
A61M

Le présent rapport a été établi pour toutes les revendications

| Lieu de la recherche | Date d'achèvement de la recherche | Examinateur |
|---|---|---|
| La Haye | 28 novembre 2022 | Bazzanini, Rita |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

page 1 de 2

**Europäisches Patentamt**
**European Patent Office**
**Office européen des brevets**

# RAPPORT DE RECHERCHE EUROPEENNE

Numéro de la demande

EP 22 18 5681

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | ES 2 467 566 A1 (LAINCO S A [ES]) 12 juin 2014 (2014-06-12) * page 1, lignes 7-29 * * page 2, lignes 26-29 * * page 5, ligne 31 – page 6, ligne 3 * * page 8, lignes 4-25 * * page 9, lignes 26-31 * ----- | 1-15 | |
| Y,D | US 3 917 821 A (MANES MILTON) 4 novembre 1975 (1975-11-04) * colonne 1, lignes 22-30 * * colonne 2, lignes 3-27 * * page 1; revendication 1 * ----- | 1-15 | |
| Y | WO 2004/043453 A1 (WELSER JENNIFER L [US]) 27 mai 2004 (2004-05-27) * alinéas [0016] – [0018] * * figure 1 * ----- | 1-15 | |

**DOMAINES TECHNIQUES RECHERCHES (IPC)**

Le présent rapport a été établi pour toutes les revendications

| Lieu de la recherche | Date d'achèvement de la recherche | Examinateur |
|---|---|---|
| La Haye | 28 novembre 2022 | Bazzanini, Rita |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.........................................................................
& : membre de la même famille, document correspondant

EPO FORM 1503 03.82 (P04C02)

page 2 de 2

## ANNEXE AU RAPPORT DE RECHERCHE EUROPEENNE
## RELATIF A LA DEMANDE DE BREVET EUROPEEN NO.

EP 22 18 5681

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

28-11-2022

| Document brevet cité au rapport de recherche | Date de publication | Membre(s) de la famille de brevet(s) | | Date de publication |
|---|---|---|---|---|
| ES 2467566 A1 | 12-06-2014 | AUCUN | | |
| US 3917821 A | 04-11-1975 | AUCUN | | |
| WO 2004043453 A1 | 27-05-2004 | AU | 2003285201 A1 | 03-06-2004 |
| | | CA | 2505092 A1 | 27-05-2004 |
| | | CN | 1717231 A | 04-01-2006 |
| | | EP | 1558236 A1 | 03-08-2005 |
| | | JP | 2006514008 A | 27-04-2006 |
| | | KR | 20050086438 A | 30-08-2005 |
| | | MX | PA05004949 A | 19-08-2005 |
| | | US | 2004091518 A1 | 13-05-2004 |
| | | US | 2006141013 A1 | 29-06-2006 |
| | | WO | 2004043453 A1 | 27-05-2004 |

EPO FORM P0460

Pour tout renseignement concernant cette annexe : voir Journal Officiel de l'Office européen des brevets, No.12/82

RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3917821 A

- WO 2004043453 A1

**Littérature non-brevet citée dans la description**

- **BURKITT JAMIE M. et al.** Effects of Oral Administration of a Commercial Activated Charcoal Suspension on Serum Osmolality and Lactate Concentration in The Dog. *JOURNAL OF VETERINARY INTERNAL MEDICINE,* 01 Septembre 2005, vol. 19 (5), 683-686